# EUROPEAN PATENT APPLICATION

(11) **EP 4 672 254 A1**
(43) Date of publication of application: **31.12.2025**
(21) Application number: 24718268.6
(22) Date of filing: 20.02.2024
(51) Int. Cl.: G16H 10/60, G16H 40/63, G16H 40/67, G06F 21/42, H04L 9/40

(54) **SMART SCALE**

(30) Priority: 22.02.2023 PT 2023118528; 16.10.2023 PT 2023118985
(71) Applicant: DAGEC - Domótica em Análise de Gestão e Contabilidade, Unipessoal Lda, 4405-617 Vila Nova De Gaia (PT)
(72) Inventor: SEQUEIRA, Pedro, 4405-617 Vila Nova De Gaia (PT)
(74) Representative: Pereira da Cruz, Joao
(86) International application number: PCT/IB2024/051615
(87) International publication number: WO 2024/176110

(57) **Abstract**

The present invention is related to electronic health equipment, particularly with multifunctional systems for measuring health indicators. The object of the present invention is an intelligent health indicator measurement system that comprises a health indicator measurement unit, configured to acquire at least one health indicator from a user; a user identification unit, configured to acquire the user's identity; a central control unit, comprising processing means configured to associate the at least one health indicator acquired by the health indicator measurement unit with the user's identity acquired by the user identification unit through the execution of a two-factor authentication process. This solution allows for the measurement of health indicators to be securely, reliably, and authentically linked to a person's identity.

## Description

### FIELD OF THE INVENTION

The present invention is related to electronic health equipment, in particular to multifunctional systems for measuring health indicators.

### BACKGROUND OF THE INVENTION

Health is a state of complete physical, mental, and social well-being that needs to be monitored, especially in increasingly ageing populations. Monitoring the health status of an individual begins with the measurement of several health indicators (weight, height, body mass index - BMI, blood pressure, among others) which can be done during a medical consultation under the supervision of health professionals, or at pharmacies having equipment for this purpose. Although medical consultation is the best way to monitor one's health status, the use of specialized equipment at pharmacies or at home is more accessible and allows a more frequent monitoring without incurring high costs.

There are several solutions disclosed in the prior art for measuring health indicators outside the context of a medical consultation, involving equipment that measures the main health indicators (weight, height, BMI) and provides the user with a printed record indicating the measurements taken. This equipment is commonly located in pharmacies; however, the measurement of health indicators is performed in a way that is dissociated from the person's identity, which diminishes the confidence that the measurements presented on a printed record correspond to the holder of said printed record.

Although there is some equipment that perform measurements of health indicators and that associate the measurements taken with the user's identification data, this equipment allows the use of unofficial identification documents (customer commercial cards, driving licenses, among others) and do not have means of authenticating the user's identity.

The present invention allows associating the measurement of health indicators with the person's identity in a secure, reliable, and authenticated manner. This authentication provides the user with a highly reliable general health status history which can be presented to health professionals, during a medical consultation, and can also be used in other contexts where a general health status is relevant, such as in insurance contracting or in the exercise of professional activities which require proven good physical fitness.

### SUMMARY OF THE INVENTION

The object of the present invention consists of an intelligent health indicator measurement system (1) which comprises:
- a health indicator measurement unit (20), configured to acquire at least one health indicator from a user;
- a user identification unit (10), configured to acquire the identity of the user;
- a central control unit (30), comprising processing means configured to associate the at least one health indicator acquired by the health indicator measurement unit with the identity of the user acquired by the user identification unit;
characterized in that the central control unit (30) is configured to validate the user's identity through the execution of a two-factor authentication process.

The authentication process allows the validation of the user's identity and allows to securely associate the health indicator measurements with said validated and authenticated identity, so as to link the measured values of the various health indicators to the identity validated and authenticated by the central control unit (30), being required the validation of the user's identity in order to perform the desired health indicator measurements.

The two-factor authentication process includes a combination of the following factors:
- user identification parameters;
- PIN code or password;
- certified digital signature methods, e.g. Digital Mobile Key;
- confirmation code sent to the user's device (e.g. smartphone) via SMS; or
- security token generated by an authentication application.

In an advantageous aspect of the present invention, the user identification unit comprises a reading module (100) adapted to read a user's physical record and extract at least one user identification parameter from the physical user record.

The physical user records consist of physical personal identification documents which are officially certified, such as Citizen Cards, passports, and other official identification documents for individuals with foreign nationalities. The extraction of user identification parameters from authenticated and officially certified physical user records allows the determination of the user's identity in a secure and unequivocal manner. Thereby, it is incompatible with the present invention the use of other documents which are not intended for personal identification, such as driving licenses, bank cards, commercial loyalty cards, among others. Thus, the system requires the use of identification documents that are inherently personal and non-transferable, making it difficult to use the system in a fraudulent manner, because that scenario would imply the unlawful possession of third-party identification documents.

The user identification parameters extracted from the physical user record include user information such as full name, height, photograph, date of birth, gender, civil identification number, tax identification number, National Health Service (NHS) user number or social security identification number. The various identification parameters extracted from the user's physical record constitute personal and non-transferable information, allowing for a secure authentication of the user's identity.

The health indicator measurement unit of the present invention includes a weighing module (200), configured to acquire the weight of the user, and a height measurement module (205), configured to acquire the height of the user.

It is also an object of the present invention a method for operating the intelligent system for measuring general health indicators that comprises the following steps:
a) user identification unit (10) acquires the user's identification parameter;
b) central control unit (30) executes a two-factor authentication process to validate the user's identity;
c) health indicator measurement unit (20) acquires at least one health indicator of the user.

The operation method of the system guides the user to measure their health indicators in a sequential manner, firstly by acquiring one or more identification parameters, followed by the two-factor authentication to validate the user's identity, and lastly, measuring one or more health indicators of the user.

### DESCRIPTION OF THE FIGURES

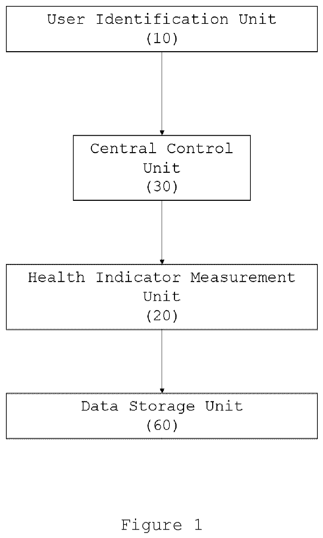

Figure 1 - Schematic representation of an intelligent health indicator measurement system (1) in accordance with the present disclosure, including a user identification unit (10), a central control unit (30), a health indicator measurement unit (20), and a data storage unit (60). The user identification unit (10) is configured to extract personal identification parameters from a physical user record, the central control unit (30) is configured to firstly validate the user's identity through the execution of a two-step authentication including the identification parameter extracted by the user identification unit (10). The two-factor authentication step precedes the acquisition of at least one health indicator of the user by the health indicator measurement unit (20), being the data acquired by the health indicator measurement unit (20) subsequently stored in the data storage unit (60), being the measured health indicators associated with the user's identity previously validated by the central control unit (30).

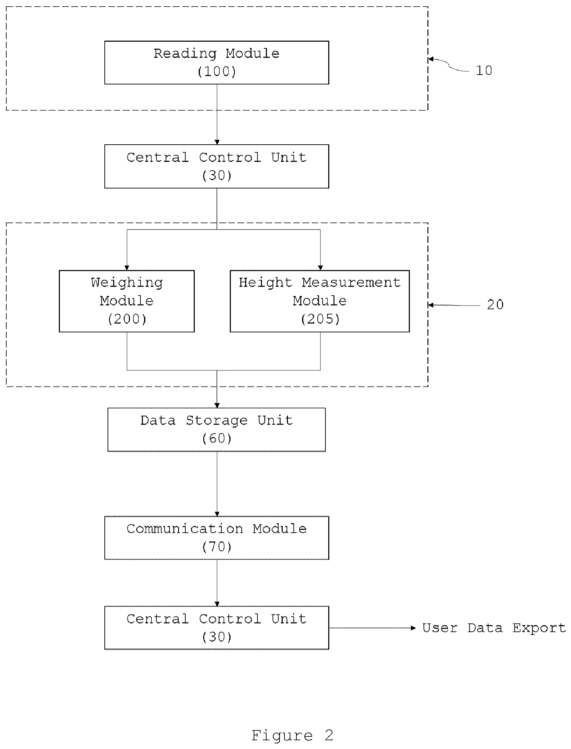

Figure 2 - Schematic representation of an intelligent health indicator measurement system (1) in accordance with the present disclosure, including a user identification unit (10) comprising a reading module (100); a central control unit (30); a health indicator measurement unit (20) comprising a weighing module (200) and a height measurement module (205); a data storage unit (60) and a communication module (70). The reading module (100) is adapted to read a physical user record and extract at least one user identification parameter from said record, such as a Citizen Card, Passport or other official identification documents. The central control unit (30) is configured to firstly validate the user's identity through the execution of a two-step authentication including the identification parameter extracted from the physical record read by the reading module (100). The two-factor authentication step precedes the acquisition of at least one health indicator of the user by the health indicator measurement unit (20), such as the acquisition of the user's weight, measured by the weighing module (200), or the acquisition of the user's height, determined by the height measurement module (205). The data acquired by the health indicator measurement unit (20) is subsequently stored in the data storage unit (60), remaining associated with the user's identity previously validated by the central control unit (30). Communication between the data storage unit (60) and the central control unit (30) is executed by the action of the communication module (70). The central control unit (30) facilitates the export of data related to the measured health indicators associated with the user's identity, being the format of the data export variable (paper, via web, via mobile applications, among others).

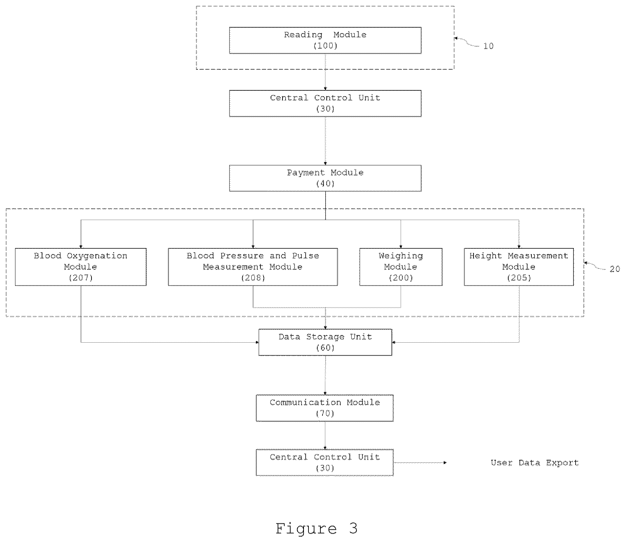

Figure 3 - Schematic representation of an intelligent health indicator measurement system (1) in accordance with the present disclosure, including a user identification unit (10) comprising a reading module (100); a central control unit (30); a health indicator measurement unit (20) comprising a weighing module (200), a height measurement module (205), a blood oxygenation module (207), and a blood pressure and pulse measurement module (208); a data storage unit (60) and a communication module (70).

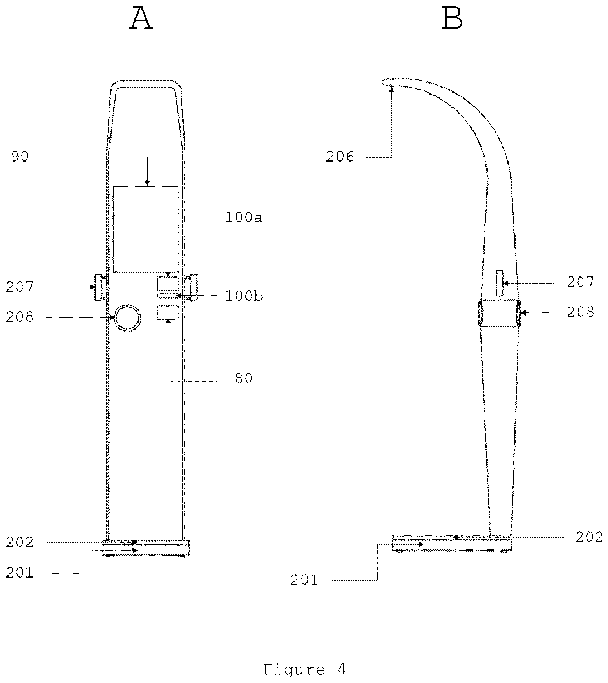

Figure 4 - Preferred embodiment of the intelligent health indicator measurement system (1) in accordance with the present disclosure. The front view (A) of the system presents a weighing module (200) which includes a base (201) and a weighing platform (202); a reading module (100) that comprises a Citizen Card reading module (100a) and passport module (100b); a blood oxygenation measurement module (207); a blood pressure and pulse measurement module (208); a printing module (80) and a display and interface device (90). Additionally, in the side view (B) of the system, the electronic sensor (206) of the height measurement module (205) is shown. The display and interface device (90) allows the user to operate the system (1) and the external configuration of the system (1) enables the user, by stepping onto the base (201) and assuming a vertical position, to access the various health indicator measurement modules.

### DETAILED DESCRIPTION OF THE INVENTION

Further details of the intelligent health indicator measurement system (1) of the present disclosure are described below.

The system may be configured so that the weighing module (200) comprises a base (201), a weighing plate (202) and an electronic device (203) having a plurality of load cells (204), so that said load cells (204) are activated by the user's weight during their use of the system. The high sensitivity of the load cells (204) allows for the user's weight to be measured with precision, having an error margin up to 0.1 kg.

The system may be configured so that the height measurement module (205) includes an electronic sensor (206) configured to determine the user's height during their use of the system. The electronic sensor (206) is configured to allow the measurement of the user's height when the user adopts an orthostatic position on the weighing plate (202).

The health indicator measurement unit (20) may comprise a blood oxygenation measurement module (207), which consists of a pulse oximeter configured to measure the user's blood oxygen saturation levels in a non-invasive manner.

The health indicator measurement unit (20) may comprise a blood pressure and pulse measurement module (208), configured to measure the user's blood pressure.

The system may include a data storage unit (60) configured to store health indicator measurement data based on the user's identity determined by the user identification unit, being said data storage unit housed on a web server. The integration of a data storage unit allows each measurement performed by the health indicator measurement unit (20) to be associated with the user's identity determined by the user identification unit (10), so that each user can consult their personal history of measured values over time.

The system may include a communication module (70) configured to establish a data communication between the central control unit (30) and the web server, thus allowing the export of data measured by the system.

The system may include a payment module (40) configured to operate according to at least one of the following payment methods: bank card, cash, mobile payment.

The system may comprise a voice control unit (50), configured to allow the control and operation of the system through voice commands voiced by the user. The voice control unit (50) allows users to control and operate the intelligent system using only voice commands without the need for tactile interfaces, such as buttons or touch screens, thus facilitating the use of the system by users who have visual or motor disabilities.

The system may include a printing module (80) configured to print data relating to the use of the system and payment data made through the payment module (40), providing the user with a paper record of the data measured by the health indicator measurement unit (20) and also payment receipts.

### EMBODIMENTS

Figure 4 presents a preferred embodiment of the present intelligent weighing and general health indicator measurement system (1).

The intelligent health indicator measurement system (1) is integrated into a vertical structure, in which the different units and their respective modules are arranged to facilitate user interaction with the system. Thus, the weighing module (200) is integrated into a step that the user must step onto, consisting of the base (201) and the weighing plate (202), with the electronic device (203) which has a plurality of load cells (204) located inside the step under the weighing plate (202), being activated when the user adopts an orthostatic position on the weighing plate (202).

Preferably, the electronic sensor (206) of the height measurement module (205) is positioned at the top of the vertical structure, to enable the correct measurement of the height of users positioned on the step of the structure adopting an orthostatic position.

In an advantageous aspect of the present invention, the display and interface device (90) is located on the inner facet of the vertical structure, at a suitable height for observation and interaction by the user when they are positioned on the step of the structure. Preferably, the display and interface device (90) consists of a touchscreen that allows for user viewing and manual input.

In a preferred embodiment, the reading module (100) is located on the inner facet of the vertical structure. Preferably, the user identification unit (10) is comprised of Citizen Card reading modules (100a) and passport modules (100b), being these modules configured to receive the physical user records to extract the necessary user identification parameters for two-factor authentication.

Preferably, the intelligent health indicator measurement system (1) also comprises a blood oxygenation module (207), located on the side face of the vertical structure, being easily accessible by the user.

The intelligent health indicator measurement system (1) may comprise a blood pressure and pulse measurement module (208) integrated into a hole adapted to the thickness of the user's arm to enable the measurement of blood pressure and pulse.

Preferably, the intelligent health indicator measurement system (1) has a printing module configured to print data relating to the use of the system, providing the user with a paper record of the data measured by the health indicator measurement unit.

The preferred method for operating the intelligent weighing and general health indicator measurement system (1) comprises the following sequential steps:
1. The user inserts their physical user record (Citizen Card or passport) into the reading module (100);
2. A welcome message and data transfer authorization under the General Data Protection Regulation (GDPR) appears on the display and interface device (90);
3. If the user refuses the conditions - the general health indicator measurement service cannot be provided;
4. If the user accepts the conditions - access to the next menu of two-factor authentication;
5. The user enters their mobile phone contact (factor #1) and waits to receive authentication instructions on their mobile phone;
6. After receiving a 4-digit code on their mobile phone, the user must enter it on the display and interface device (90);
7. Next menu: Clinical data entry (Message "Do you have any known health complications?") - Access to a selection matrix of 1 or more diseases;
8. Next menu: selection of payment method;
9. Measurement of general health indicators: follow instructions displayed on the display and interface device (90);
10.Next menu: The user selects whether to print on paper or send data to their mobile phone.
11. Thank you message: "THANK YOU, AND SEE YOU SOON!".

The present invention should not be limited to the embodiments described in this document, as various alterations and combinations that remain within the scope of the present invention are possible.

## Claims

1. An intelligent system for measuring health indicators comprising:
a. a user identification unit, configured to acquire the identity of the user;
b. a health indicator measurement unit, configured for the acquisition of at least one health indicator of a user;
c. a central control unit, comprising processing means configured to associate the at least one health indicator acquired by the health indicator measurement unit with the user's identity acquired by the user identification unit;
**characterized in that** the central control unit is configured to validate the user's identity through the execution of a two-factor authentication process.

2. An intelligent system for measuring health indicators according to the previous claim **characterized by** the two-factor authentication process involving the combination of the following factors: user identification parameter, PIN code, Digital Mobile Key, confirmation code sent to the user's device, security token generated by an authentication application.

3. An intelligent system for measuring health indicators according to any of the previous claims **characterized by** the user identification unit comprising a reading module adapted to read a physical user record and extract at least one user identification parameter from the physical user record.

4. An intelligent system for measuring health indicators according to the previous claim **characterized by** the user identification parameters being as follows: name, height, photograph, date of birth, gender, civil identification number, tax identification number, National Health Service (NHS) user number or Social Security identification number.

5. An intelligent system for measuring health indicators according to any of the previous claims **characterized by** the health indicator measurement unit comprising a weighing module, configured to acquire the user's weight; and a height measurement module, configured to acquire the user's height.

6. An intelligent system for measuring health indicators according to the previous claims **characterized in that** the weighing module comprises a base, a weighing plate and an electronic device containing a plurality of load cells, so that the load cells are activated by the user's weight.

7. An intelligent system for measuring health indicators according to the previous claims **characterized in that** the height measurement module comprises an electronic sensor configured to determine the user's height.

8. An intelligent system for measuring health indicators according to the previous claims **characterized by** comprising a payment module in which said payment module is configured to operate according to at least one of the following payment methods: bank card, cash, mobile payment.

9. An intelligent system for measuring health indicators according to the previous claims **characterized by** comprising a voice control unit, configured to allow control and operation of the system through voice commands voiced by the user.

10. An intelligent system for measuring health indicators according to claims 1 - 8 **characterized in that** the health indicator measurement unit comprises a blood oxygenation measurement module consisting of a pulse oximeter configured to measure the user's blood oxygen saturation levels.

11. An intelligent system for measuring health indicators according to claims 1 - 8 **characterized in that** the health indicator measurement unit comprises a blood pressure and pulse measurement module, configured to measure the user's blood pressure.

12. An intelligent system for measuring health indicators according to the previous claims **characterized in that** the system comprises a data storage unit configured to store health indicator measurement data based on the user's identity.

13. An intelligent system for measuring health indicators according to the previous claim **characterized in that** the data storage unit is housed on a web server and that the system comprises a communication module configured to establish a data communication between the central control unit and the web server.

14. A method for operating the intelligent system for measuring health indicators in accordance with any one of the previous claims comprising the following steps:
a. the user identification unit acquires the user identification parameter;
b. the central control unit executes a two-factor authentication process to validate the user's identity;
c. the health indicator measurement unit acquires at least one health indicator from the user.
